# EUROPÄISCHE PATENTSCHRIFT

(11) **EP 2 790 790 B1**
(45) Veröffentlichungstag und Bekanntmachung des Hinweises auf die Patenterteilung: **19.04.2017**
(21) Anmeldenummer: 12781093.5
(22) Anmeldetag: 05.11.2012
(51) Int. Cl.: A61K 8/34, A61K 8/39, A61K 8/92, A61K 8/04, A61K 8/81, A61K 8/86, A61Q 5/10

(54) **OXIDATIONSFÄRBEMITTEL AUF GELBASIS AUS EMULGATORKOMBINATION UND VERDICKER**
GEL-BASED OXIDATION COLORANT COMPRISING AN EMULSIFIER COMBINATION AND THICKENER
COLORANT D'OXYDATION SOUS FORME DE GEL CONSTITUÉ D'UNE ASSOCIATION D'ÉMULSIFIANTS ET D'UN ÉPAISSISSANT

(30) Priorität: 16.12.2011 DE 102011088819
(43) Veröffentlichungstag der Anmeldung: 22.10.2014
(73) Patentinhaber: Henkel AG & Co. KGaA, 40589 Düsseldorf (DE)
(72) Erfinder: WITTE, Christiane, 25491 Hetlingen (DE); MANNECK, Hartmut, 23858 Barnitz (DE)
(86) Internationale Anmeldenummer: PCT/EP2012/071796
(87) Internationale Veröffentlichungsnummer: WO 2013/087293

(56) Entgegenhaltungen:
- EP-A1- 1 634 572
- EP-A2- 2 340 807
- WO-A1-88/00823
- WO-A1-97/01323
- WO-A1-2005/067874
- WO-A1-2006/015650
- DE-A1-102006 005 768

## Beschreibung

Gegenstand der Erfindung ist ein Mittel zum oxidativen Färben von keratinischen Fasern, welches sich durch eine gelförmige Basis aus einer spezifischen Kombination von Emulgatoren und einen polymeren Verdicker auszeichnet.

Die Veränderung von Form und Farbe der Haare stellt einen wichtigen Bereich der modernen Kosmetik dar. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für Haare, kennt der Fachmann je nach Anforderungen an die Färbung diverse Färbesysteme. Für permanente, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Oxidative Haarfärbemittel sind jedoch trotz ihrer vorteilhaften Färbeeigenschaften für den Anwender mit Nachteilen behaftet.

Erstens führen der Einsatz der Oxidationsmittel zur Entwicklung der eigentlichen Färbung und der zur Ausfärbung notwendige basische pH-Wert der Mittel zu Schädigungen in der Haarstruktur und auf der Haaroberfläche. Andererseits besteht weiterhin Bedarf nach Mitteln mit verbesserten Färbeleistungen. Insbesondere Mittel für verbesserte, gleichmäßige Färbungen mit hoher Intensität und Farbigkeit sowie gutem Grauabdeckungsvermögen, insbesondere auf unterschiedlich vorbehandelten Haaren, sind weiterhin im Focus der Entwicklung. Da sowohl Färbeergebnis wie auch damit einhergehende Haarfaserbelastung von der Einwirkzeit des Mittels abhängen, besteht das Bedürfnis, den Färbefortschritt während des Färbevorgangs nachverfolgen zu können und so den Zielpunkt der Behandlung genau zu bestimmen, um neben dem gewünschten Färbeergebnis auftretende Haarfaserbelastungen zu minimieren. Dazu ist es wünschenswert, eine transparente oder klare Färbezubereitung bereitzustellen.

Schließlich hat sich gezeigt, dass gängige Haarfärbemittel aufgrund der Vielzahl der verwendeten Inhaltsstoffe häufig unbefriedigende rheologische Eigenschaften besitzen. Diese drücken sich insbesondere in Viskositätsschwankungen während der Lagerung und der Anwendung aus. So sind Mittel, die im Verlauf der Lagerung ausdicken, mit Problemen bei Entnahme, Dosierung und Anwendung verbunden, während Mittel, die eine Viskositätserniedrigung während der Lagerung erfahren, ebenfalls ungenügende Anwendungseigenschaften besitzen.

Aufgabe der vorliegenden Erfindung ist es daher, die oben genannten Nachteile oxidativer Haarfärbemittel herabzusenken. Die Färbemittel sollen durch eine genaue Endpunkterkennung der Behandlung eine verringerte Schädigung des Haares bewirken. Insbesondere Schutz vor oxidativen Schädigungen der Haarstruktur und der Haaroberfläche soll durch die Haarfärbemittel erzielt werden. Die Färbemittel sollen gleichzeitig über verbesserte rheologische Eigenschaften verfügen, die sich insbesondere in verbesserter Viskositätsstabilität und Scherempfindlichkeit ausdrücken. Weiterhin soll das Färbemittel eine verbesserte Farbintensität und eine verbesserte Grauabdeckung gegenüber herkömmlichen Färbemitteln ermöglichen.

In diesem Zusammenhang beschreibt WO 2005/067874 A1 transparente, gelförmige Mittel zur oxidativen Haarfärbung, mit einem bestimmten Peroxidstabilisator, einem bestimmten Polymerverdicker und Wasser.

In nicht vorhersehbarer Weise wurde nun gefunden, dass eine neuartige Färbezubereitungsgrundlage auf Basis eines gelförmigen Trägers aus einer Emulgator-Kombination von bestimmten nichtionischen, polyethoxylierten Emulgatoren (i), und bestimmten Polyethylenglycol(en) (ii) mit einem mittleren Molekulargewicht von 100 bis 100000 g Mol⁻¹ und einem polymeren Verdicker, die Bereitstellung von transparenten Färbemitteln mit hoher Viskositätsstabilität für gleichzeitiger Färbefortschrittsbeobachtung ermöglicht.

Erster Gegenstand der vorliegenden Erfindung ist daher ein zum Färben keratinischer Fasern, insbesondere menschlicher Haare, welches in einem gelförmigen Träger mindestens ein Oxidationsfarbstoffvorprodukt enthält und welches dadurch gekennzeichnet ist, dass das Mittel frei von Fettstoffen ist, und der gelförmige Träger des Färbemittels
a) mindestens eine Emulgator-Kombination aus
   i) einem oder mehreren nichtionischen, polyethoxylierten Emulgatoren (i), ausgewählt aus ethoxylierten Fettalkoholen mit 8 bis 22 Kohlenstoffatomen und/oder ethoxylierten Rizinusöl und
   ii) einem oder mehreren Polyethylenglycol(en) (ii) mit einem mittleren Molekulargewicht von 100 bis 100000 g Mol⁻¹
b) und mindestens einen polymeren Verdicker, ausgewählt aus Homopolymeren von Acrylsäure oder Methacrylsäure und/oder Copolymeren von Acrylsäure oder Methacrylsäure mit C₁-C₄-Alkylestern von Acrylsäure oder Methacrylsäure,
enthält.

Die erfindungsgemäßen Mittel zeichnen sich durch gute Färbeeigenschaften bei gleichzeitigem Schutz vor Schädigungen für das menschliche Haar aus. Insbesondere lassen sich mit den erfindungsgemäßen Mitteln gleichmäßige Färbungen mit hoher Farbintensität und guter Echtheit erzielen. Schließlich besitzen die erfindungsgemäßen Mittel signifikante Vorteile hinsichtlich der Viskositätsstabilität bei Lagerung und Anwendung. Die Färbezubereitungen zeichnen sich weiterhin durch eine optische Transparenz aus, die es ermöglicht, den Farbentwicklungsfortschritt visuell nachverfolgen zu können.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Farbstoffvorprodukte in einem gelförmigen Träger. Dieser Träger ist bevorzugt optisch transparent. Besonders bevorzugt erhält der gelförmige Träger seine optische Transparenz auch nach Vermischen mit einer Oxidationsmittelzubereitung zu einem anwendungsbereiten Oxidationsfärbemittel. Optische Transparenz ist dabei erfindungsgemäß als die visuelle Erkennbarkeit der Farbentwicklung auf der Haarfaser nach Auftragen des Färbemittels zu verstehen. Üblicherweise liegt die resultierende Schichtdicke der Färbezubereitung auf der Haarfaser bei 0,00001 mm bis 0,1 mm.

Optisch transparente Gele können daher auch mit dem Begriff "klare gelförmige Träger" umschrieben werden. Dazu liegen die Mittel nicht als Emulsion oder Lipo-/Oleogel vor. Die Mittel enthalten daher keine Fettkörper und sind frei von Fettstoffen, insbesondere frei von Fettalkoholen, hydrophoben Fettsäureestern sowie mineralischen Fettstoffen. Unter dem Begriff "frei von Fettstoffen" ist dabei ein Anteil von höchstens 0,5 Gew.-%, bevorzugt höchstens 0,1 Gew.-% und weiter bevorzugt höchstens 0,01 Gew.-%, jeweils bezogen auf das Gesamtgewicht des Mittels zu verstehen.

Fettstoffe im Sinne der Erfindung zeichnen sich dabei durch eine Wasserlöslichkeit kleiner als 0,1 g/L bei Normalbedingungen aus.

Fettalkohole sind dabei lineare oder verzweigte, gesättigte oder ungesättigte Alkan-1-ole mit 8 bis 24 Kohlenstoffatomen, wie Cetylalkohol, Stearylalkohol oder Oleylalkohol.

Hydrophobe Fettsäureester sind dabei Fettsäuretriglyceride ohne zusätzliche hydrophile Strukturelemente, wie in Olivenöl oder Cocosfett, Fettsäurediester mit Diolen, wie Ethylenglycoldistearat, und Fettsäureester mit Alkanolen, wie Isopropylmyristat oder Jojobaöl.

Mineralische Fettstoffe sind dabei Kohlenwasserstoffe wie Polyethylene, Vaseline oder Paraffin.

Der gelförmige Träger ist im Sinne der Erfindung ist wässrig, alkoholisch oder wässrig-alkoholisch.

Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Mittel. Erfindungsgemäß bevorzugt sind wässrige Träger, so dass das Mittel einen Anteil von mindestens 80 Gew.-%, bevorzugt mindestens 85 Gew.-%, bezogen auf das Gesamtgewicht des Mittels aufweist.

Als gelförmig ist erfindungsgemäß ein formbeständiges, leicht deformierbares, an Flüssigkeiten reiches disperses System aus mindestens zwei Komponenten zu verstehen, die aus einem festen, kolloidzerteilten Stoff mit langen oder stark verzweigten Teilchen als Verdickungsmittel und einer Flüssigkeit (meist Wasser) als Dispersionsmittel bestehen. Dabei ist die feste Substanz kohärent, d. h. sie bildet im Dispersionsmittel ein räumliches Netzwerk.

Als ersten wesentlichen Inhaltsstoff umfasst das erfindungsgemäße Mittel in dem gelförmigen Träger mindestens eine Emulgator-Kombination aus
i) einem oder mehreren nichtionischen, polyethoxylierten Emulgatoren (i), ausgewählt aus ethoxylierten Fettalkoholen mit 8 bis 22 Kohlenstoffatomen und/oder ethoxylierten Rizinusöl und
ii) einem oder mehreren Polyethylenglycol(en) (ii) mit einem mittleren Molekulargewicht von 100 bis 100000 g·Mol⁻¹.

In einer Ausführungsform sind die nichtionischen Emulgatoren dabei ausgewählt aus ethoxylierten, linearen Fettalkoholen, bevorzugt einer Kettenlänge mit 8 bis 22 Kohlenstoffatomen. Unter einem ethoxylierten Fettalkohol wird im Sinne der Erfindung ein Anlagerungsprodukt von Ethylenoxid an einen Fettalkohol verstanden, wobei der Ethoxylierungsgrad die Molmenge Ethylenoxid (EO) angibt, die durchschnittlich pro Mol Fettalkohol angelagert wurde. Bevorzugte ethoxylierte Fettalkohole sind Ethylenoxid-Anlagerungsprodukte an Caprinalkohol, Laurylalkohol, Isotridecylalkohol, Myristylalkohol, Cetylalkohol, Palmoleylalkohol, Stearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Besonders bevorzugt sind Anlagerungsprodukte an technische Fettalkohole bzw. deren Mischungen mit 12 bis 18 Kohlenstoffatomen wie beispielsweise Kokos-, Palm-, Palmkern- oder Talgfettalkohol, insbesondere Kokos- und/ oder Talgfettalkohol.

Je nach Herstellungsmethode fallen die erfindungsgemäßen ethoxylierten Fettalkohole als ein Gemisch mit einer unterschiedlichen Ethoxylierungsgradverteilung an. Im Sinne der Erfindung werden diese Emulgatoren daher nach dem durchschnittlichen Ethoxylierungsgrad gekennzeichnet. Dieser ist üblicherweise als Ziffer hinter dem Fettalkohol-Suffix "eth-" in der INCI-Bezeichnung erkennbar. Besonders geeignete ethoxylierte Fettalkohole sind Fettalkohole mit einem Ethoxylierungsgrad wird von 12 bis 100, bevorzugt 20 bis 80 Mol Ethylenoxid pro Mol Fettalkohol verstanden. Beispiele sind Ceteaeth-12, Ceteth-15, Ceteareth-15, Laneth-16, Ceteth-16, Oleth-16, Steareth-16, Oleth-20, Ceteth-20, Ceteareth-20, Ceteareth-23, Laureth-23, Ceteareth-25, Ceteareth-30, Ceteth-40, Laneth-40, Oeth-50, Ceteareth-50, Ceteareth-60, Ceteareth-80.

In einer weiteren Ausführungsform sind die nichtionischen Emulgatoren dabei ausgewählt aus ethoxylierten Rizinusöl. Hierzu zählt erfindungsgemäß ethoxyliertes, gehärtetes (d.h. hydriertes) und ungehärtetes Rizinusöl. Der Ethoxylierungsgrad gibt die Molmenge Ethylenoxid (EO) an, die durchschnittlich pro Mol Rizinusöl angelagert wurde. Bevorzugte ethoxylierte Rizinusöle sind die unter INCI-Bezeichnungen bekannten PEG-5 Castor Oil, PEG-7 hydrogenated Castor Oil, PEG-10 hydrogenated Castor Oil, PEG-25 hydrogenated Castor Oil, PEG-35 Castor Oil, PEG-36 Castor Oil, PEG-40 Castor Oil, PEG-40 hydrogenated Castor Oil, PEG-50 hydrogenated Castor Oil, PEG-60 Castor Oil, PEG-60 hydrogenated Castor Oil, PEG-80 Castor Oil, PEG-80 hydrogenated Castor Oil, PEG-100 Castor Oil, PEG-100 hydrogenated Castor Oil, PEG-120 Castor Oil, PEG-120 hydrogenated Castor Oil, PEG-150 Castor Oil, PEG-150 hydrogenated Castor Oil, PEG-200 Castor Oil, PEG-200 hydrogenated Castor Oil.

Bevorzugt als nichtionische Emulgatoren sind Rizinusöle mit einem durchschnittlichen Ethoxylierungsgrad von 20 bis 150, bevorzugt 30 bis 100 und besonders bevorzugt 35 bis 80. Besonders bevorzugt ist PEG-40 hydrogenated Castor Oil.

Die nichtionischen, polyethoxylierten Emulgatoren (i) sind in einer Ausführungsform im erfindungsgemäßen Mittel 0,1 bis 10,0 Gew.-%, bevorzugt 0,3 bis 8,0 Gew.-%, besonders bevorzugt 0,5 bis 5,0 Gew.-% und insbesondere 0,8 bis 3,0 Gew.-%, bezogen auf das Gesamtgewicht des Mittels, enthalten.

Als weitere wesentliche Komponente umfasst Emulgator-Kombination mindestens einen oder mehrere Polyethylenglycol(e) (ii) mit einem mittleren Molekulargewicht von 100 bis 100000 g·Mol⁻¹.

In einer Ausführungsform sind dabei Polyethylenglycol(e) (ii) bevorzugt, die ein mittleres Molekulargewicht von 500 bis 15000 g·Mol⁻¹, bevorzugt 1000 bis 10000 g·Mol⁻¹ und besonders bevorzugt 1200 bis 5000 g·Mol⁻¹ besitzen. Beispiele für geeignete Polyethylenglycole sind PEG-30, PEG-32, PEG-35, PEG-38, PEG-40, PEG-45, PEG-50, PEG-60, PEG-75, PEG-80, PEG-100 (d.h. Ethoxylierungsgrad 30, 32, 35, 38, 40, 50 etc.) oder PEG 1000, PEG 1200, PEG 1500, PEG 2000, PEG 3000, PEG 5000, PEG 6000 (d.h. mittleres Molgewicht 1000, 1200, 1500, 2000 etc.).

Erfindungsgemäße Mittel enthalten die Polyethylenglycol(e) (ii) bevorzugt zu 0,1 bis 10,0 Gew.-%, bevorzugt 0,3 bis 8,0 Gew.-%, besonders bevorzugt 0,5 bis 5,0 Gew.-% und insbesondere 0,8 bis 3,0 Gew.-%, bezogen auf das Gesamtgewicht des Mittels.

Weiterhin hat es sich gezeigt, dass für besonders gute Transparenz die erfindungsgemäßen Mittel gelförmige Träger mit einer Emulgator-Kombination aus nichtionischen, polyethoxylierten Emulgatoren (i) und Polyethylenglycolen (ii) in einem bestimmten Gewichtsverhältnis zueinander enthalten.

Eine Ausführungsform des ersten Erfindungsgegenstands ist daher dadurch gekennzeichnet, dass die Emulgator-Kombination a) ein Gewichtsverhältnis aus dem Anteil der nichtionischen, polyethoxylierten Emulgatoren (i) und dem Anteil des Polyethylenglycols (ii) einen Wert von 3:1 bis 1:3, bevorzugt von 2:1 bis 1:2 und insbesondere von 1,5:1 bis 1:1,5, aufweist.

Durch den Gesamtanteil der Emulgatorkombination a) im erfindungsgemäßen Mittel lässt sich einerseits Fließvermögen der Mittel und Gelstabilität einerseits steuern und andererseits die Löslichkeit der übrigen Inhaltstoffe im Mittel positiv beeinflussen und so Eintrübungen durch dispergierte Inhaltstoffe reduzieren. Bevorzugt sind Anteile von 0,5 bis 15,0 Gew.-%, bevorzugt 1,0 bis 10,0 Gew.-%, besonders bevorzugt 1,5 bis 7,5 Gew.-% und insbesondere 3,0 bis 5,0 Gew.-%, bezogen auf das Gesamtgewicht des Mittels.

Eine weitere Ausführungsform des ersten Erfindungsgegenstands ist daher dadurch gekennzeichnet, dass das Mittel die Emulgator-Kombination in einem Anteil von 0,5 bis 15,0 Gew.-%, bevorzugt 1,0 bis 10,0 Gew.-%, besonders bevorzugt 1,5 bis 7,5 Gew.-% und insbesondere 3,0 bis 5,0 Gew.-%, bezogen auf das Gesamtgewicht des Mittels, enthält.

Als weiteren wesentlichen Inhaltsstoff enthalten die erfindungsgemäßen Mittel mindestens einen polymeren Verdicker, der ausgewählt ist aus Homopolymeren von Acrylsäure oder Methacrylsäure und/oder Copolymeren von Acrylsäure oder Methacrylsäure mit C₁-C₄-Alkylestern von Acrylsäure oder Methacrylsäure.

Geeignete Homopolymere von Acrylsäure sind gegebenenfalls vernetzte Polyacrylsäuren (Carbomer; Handelsbezeichnung beispielsweise Carbopol) sowie Polyacrylate als deren partiell oder vollständig deprotonierten Salz-Formen. Dabei können die sauren Gruppen ganz oder teilweise als Natrium-, Kalium-, Ammonium-, Mono- oder Triethanolammonium-Salz vorliegen. Als bevorzugte Vernetzungsagentien eignen sich beispielsweise Allylether von Pentaerythrit, von Sucrose und von Propylenglycol sowie Ethylenglycoldimethacrylat. Beispiele für Homopolymere von Methacrylsäure sind entsprechend gegebenenfalls vernetzte Polymethacrylsäuren und Polymethacrylate.

In einer weiteren Ausführungsform kann das erfindungsgemäße Mittel als polymeren Verdicker mindestens ein anionisches Acrylsäure- und/oder Methacrylsäure-Polymerisat oder -Copolymerisat enthalten. Bevorzugte Polymerisate dieser Art sind:
- Polymerisate z.B. aus wenigstens 10 Gew.-% Acrylsäure-C₁-C₄-alkylester, 25 bis 70 Gew.-% Methacrylsäure und ggf. bis zu 40 Gew.-% eines weiteren Comonomeren,
- Mischpolymerisate aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll^{®} D (BASF).
- Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Besonders bevorzugte Copolymere sind beispielsweise Copolymere aus Acrylsäure, Methacrylsäure oder deren C₁-C₄-Alkylestern, wie sie unter der INCI-Deklaration Acrylates Copolymere vertrieben werden. Bevorzugt ist dabei die Kombination aus Methacrylsäure und Ethylacrylat sowie gegebenenfalls vernetzenden, multifunktionalen Monomeren. Ein bevorzugtes Handelsprodukt dafür ist beispielsweise Aculyn^{®} 33 bzw. 33A der Firma Rohm & Haas.

Besonders gute Gelbildung und stabile Viskositätseinstellung einerseits und leichte Herstellung andererseits wird jedoch bei der Verwendung von Polyacrylsäure selbst erhalten.

Eine weitere bevorzugte Ausführungsform des ersten Erfindungsgegenstands ist daher dadurch gekennzeichnet, dass das Mittel als polymeren Verdicker Polyacrylsäure (Carbomer) enthält.

Der bzw. die polymere(n) Verdicker sind in den erfindungsgemäßen Mitteln bevorzugt in einem Anteil von 0,1 bis 5,0 Gew.-%, bevorzugt 0,3 bis 3,0 Gew.-%, besonders bevorzugt 0,5 bis 2,5 Gew.-% und insbesondere 1,0 bis 2,0 Gew.-%, bezogen auf das Gesamtgewicht des Mittels, enthalten.

Ein bevorzugtes erfindungsgemäßes Mittel weist einen gelförmigen Träger auf, der 0,2 bis 2,5 Gew.-% nichtionische, polyethoxylierte Emulgatoren (i), 0,2 bis 2,5 Gew.-% Polyethylenglycole (ii) und 0,2 bis 2,0 Gew.-% polymeren Verdicker, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% Ceteareth-20, 0,2 bis 2,5 Gew.-% PEG 1500 und 0,2 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% Ceteareth-30, 0,2 bis 2,5 Gew.-% PEG 1500 und 0,2 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% Ceteareth-50, 0,2 bis 2,5 Gew.-% PEG 1500 und 0,2 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% PEG-40 hydrogenated Castor Oil, 0,2 bis 2,5 Gew.-% PEG 1500 und 0,2 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% PEG-60 hydrogenated Castor Oil, 0,2 bis 2,5 Gew.-% PEG 1500 und 0,2 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% Ceteareth-20, 0,2 bis 2,5 Gew.-% PEG 1500 und 0,2 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% Ceteareth-30, 0,2 bis 2,5 Gew.-% PEG 1500 und 0,2 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% Ceteareth-50, 0,2 bis 2,5 Gew.-% PEG 1500 und 0,2 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% PEG-40 hydrogenated Castor Oil, 0,2 bis 2,5 Gew.-% PEG 1500 und 0,2 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 0,2 bis 2,5 Gew.-% PEG-60 hydrogenated Castor Oil, 0,2 bis 2,5 Gew.-% PEG 1500 und 0,2 bis 2,0 Gew.-% Carbomer, jeweils bezogen auf das Gesamtgewicht des Mittels, enthält.

Ein bevorzugtes erfindungsgemäßes Mittel weist einen gelförmigen Träger auf, der 1,5 bis 2,5 Gew.-% nichtionische, polyethoxylierte Emulgatoren (i), 1,5 bis 2,5 Gew.-% Polyethylenglycole (ii) und 1,0 bis 2,0 Gew.-% polymeren Verdicker, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% Ceteareth-20, 1,5 bis 2,5 Gew.-% PEG 1500 und 1,0 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% Ceteareth-30, 1,5 bis 2,5 Gew.-% PEG 1500 und 1,0 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% Ceteareth-50, 1,5 bis 2,5 Gew.-% PEG 1500 und 1,0 bis 2,0 Gew.-% Acrylates Copolymer, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% PEG-40 hydrogenated Castor Oil, 1,5 bis 2,5 Gew.-% PEG 1500 und 1,0 bis 2,0 Gew.-% Acrylates Copolymer" jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% PEG-60 hydrogenated Castor Oil, 1,5 bis 2,5 Gew.-% PEG 1500 und 1,0 bis 2,0 Gew.-% Acrylates Copolymer, jeweils bezogen auf das Gesamtgewicht des Mittels, enthält.

Ein bevorzugtes erfindungsgemäßes Mittel weist einen gelförmigen Träger auf, der 1,5 bis 2,5 Gew.-% Ceteareth-20, 1,5 bis 2,5 Gew.-% PEG 1500 und 1,0 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% Ceteareth-30, 1,5 bis 2,5 Gew.-% PEG 1500 und 1,0 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% Ceteareth-50, 1,5 bis 2,5 Gew.-% PEG 1500 und 1,0 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% PEG-40 hydrogenated Castor Oil, 1,5 bis 2,5 Gew.-% PEG 1500 und 1,0 bis 2,0 Gew.-% Carbomer, jeweils bezogen auf das Gesamtgewicht des Mittels, enthält.

Ein weiteres bevorzugtes erfindungsgemäßes Mittel weist einen gelförmigen Träger auf, der 1,5 bis 2,5 Gew.-% PEG-60 hydrogenated Castor Oil, 1,5 bis 2,5 Gew.-% PEG 1500 und 1,0 bis 2,0 Gew.-% Carbomer, jeweils bezogen auf das Gesamtgewicht des Mittels, enthält.

Schließlich enthalten die erfindungsgemäßen Mittel mindestens ein Oxidationsfarbstoffvorprodukt. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp. Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Bevorzugte Entwicklerkomponenten werden ausgewählt aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxy-ethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on, sowie den physiologisch verträglichen Salzen dieser Verbindungen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäß bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diamino-phenoxy)propan, 1-Methoxy-2-amino-4-(2-hydroxyethylamino)benzol, 1,3-Bis(2,4-diaminophenyl)-propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Färbemittel, verwendet.

Weiterhin können die erfindungsgemäßen Mittel zur Nuancierung der Färbung als zusätzliche farbverändernde Komponente mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Eine weitere Ausführungsform der vorliegenden Erfindung ist daher dadurch gekennzeichnet, dass das Mittel zusätzlich mindestens einen direktziehenden Farbstoff enthält.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, HC Blue 16 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass Oxidationsfarbstoffvorprodukte oder direktziehende Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder dessen Anlagerungsprodukte an Harnstoff, Melamin sowie Natriumborat in Frage. Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Bei einer Anwendung von zusätzlichen Oxidationsmitteln wird das eigentliche Färbemittel zweckmäßigerweise unmittelbar vor der Anwendung durch Mischung einer erfindungsgemäßen Mittel, enthaltend in einem voranstehend beschriebenen gelförmigen Träger mindestens ein Oxidationsfarbstoffvorprodukt, sowie einer Zubereitung, enthaltend das zusätzliche Oxidationsmittel, insbesondere Wasserstoffperoxid, hergestellt.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Weiterhin hat es sich als vorteilhaft erweisen, wenn die Oxidationsmittelzubereitungen, insbesondere aus Oxidationsfärbemitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriamin-penta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure.

Die erfindungsgemäßen Mittel können weitere Hilfs- und Zusatzstoffe enthalten.

Erfindungsgemäße geeignete Mittel können weiterhin neben den polymeren Verdicker, ausgewählt aus Homopolymeren von Acrylsäure oder Methacrylsäure und/oder Copolymeren von Acrylsäure oder Methacrylsäure mit C₁-C₄-Alkylestern von Acrylsäure oder Methacrylsäure, zusätzlich mindestens ein weiteres Verdickungsmittel enthalten. Dadurch lässt sich sie Rheologie der erfindungsgemäßen Mittel lässt sich in die vom Verbraucher gewünschten fließfähigen Systeme überführen.

Bevorzugt handelt es sich bei dem weiteren polymeren Verdickungsmittel um einen hydrophilen Verdicker. Erfindungsgemäß einsetzbare, polymere Verdickungsmittel sind unter anderem
- synthetische Polymere, insbesondere Homo- oder Co-Polymere der Acrylsäure, ihrer Salze oder ihrer Alkylester, die kationisch oder anionisch in der Alkylkette modifiziert sind; Polyacrylamidpolymere, insbesondere Homo- oder Co-Polymere von Acrylsäureamid und/oder Methacrylsäureamid, die gegebenenfalls kationisch oder anionisch modifiziert sind; und Copolymere aus Acrylsäure und Acrylsäureamid;
- polysaccharidische Polymere, wie beispielsweise Algin, Alginate, Glucane wie Dextran, Pullulan, Curdlan, Cellulose, Laminarin, Amylose oder Lichenin, Traganth, Karaya-Gummi, Ghatti-Gummi, Agar, Carrageenan, Chitin, Chitosan, Gummi arabicum, Gellan, Carob Gummi, Galactonmannane wie Guar oder Johannisbrotkernmehl, Tamarindenkernmehl oder deren Derivate;
- anorganische Verdicker, insbesondere geeignete Elektrolyte, wie Natriumchlorid oder Kaliumchlorid, Schichtsilicate (polymere, kristalline Natriumdisilicate) und Magnesium Aluminium Silicate oder Bentonite, besonders Smektite, wie Montmorillonit oder Hectorit, die gegebenenfalls auch geeignet modifiziert sein können;
sowie deren Mischungen.

Es hat sich gezeigt, dass insbesondere verdickende, polysaccharidische Polymere milde und lagerstabile Formulierungen ergeben. Erfindungsgemäß bevorzugte polymere Verdickungsmittel sind daher verdickende, polysaccharidische Polymere. Besonders bevorzugte Polysaccharide sind dabei Polymere auf Cellulose-Basis. Hierbei können chemisch modifizierten Cellulosen, wie beispielsweise Acetyl-, Methyl- oder Ethylcellulosen, Hydroxyalkylcellulosen oder Carboxyalkylcellulosen eingesetzt werden. Besonders bevorzugte Cellulosederivate besitzen saccharidische Seitenketten, wie beispielsweise Xanthan.

Eine Ausführungsform der vorliegenden Erfindung ist dadurch gekennzeichnet, dass das Mittel als polymeres Verdickungsmittels ein polysaccharidisches Polymer, bevorzugt Xanthan, enthält.

Erfindungsgemäß bevorzugte Mittel sind dadurch gekennzeichnet, dass das zusätzlich polymere Verdickungsmittel zu 0,005 bis 1,0 Gew.-%, insbesondere zu 0,01 bis 0,5 Gew.-%, und ganz besonders zu 0,05 bis 0,3 Gew.-%, jeweils bezogen auf das Gesamtgewicht des Mittels, enthalten ist.

Weiterhin können die erfindungsgemäßen Mittel zusätzliche grenzflächenaktive Substanzen, wie anionische Tenside, zwitterionische Tenside und/oder amphotere Tenside enthalten.

Geeigneten anionische oberflächenaktiven Stoffe sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kaliumund Ammonium- sowie der Mono, Di- und Trialkanolammoniumsalze mit 2 bis 4 C-Atomen in der Alkanolgruppe,
- lineare und verzweigte Fettsäuren (Seifen),
- Ethercarbonsäuren,
- Acylsarcoside, Acyltauride und/oder Acylisethionate von Fettsäuren,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate, lineare α-Olefinsulfonate, und Sulfonate ungesättigter Fettsäuren,
- α-Sulfofettsäuremethylester von Fettsäuren,
- Alkylsulfate und Alkylethersulfate,
- Gemische oberflächenaktiver Hydroxysulfonate,
- sulfatierte Hydroxyalkylpolyethylen- und/oder Hydroxyalkylenpropylenglykolether,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen,
- ggf. ethoxylierte Alkyl- und/oder Alkenyletherphosphate,
- sulfatierte Fettsäurealkylenglykolester,
- Monoglyceridsulfate und Monoglyceridethersulfate.

Insbesondere enthalten die erfindungsgemäßen Färbemittel zusätzlich anionische Tenside, ausgewählt aus Fettsäuren, Alkylsulfaten, Alkylethersulfaten und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Erfindungsgemäß bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) vermarktet.

Die anionischen, amphoteren und zwitterionischen Tenside sind in dem erfindungsgemäßen Mittel bevorzugt in einer Gesamtmenge von 0,05 bis 30 Gew.-%, besonders bevorzugt von 0,1 bis 20 Gew.-%, jeweils bezogen auf das Gesamtgewicht des Mittels, enthalten.

Weiterhin hat es sich als vorteilhaft erwiesen, wenn die erfindungsgemäßen Mittel neben den oben beschriebenen nichtionischen Emulgatoren zusätzliche nichtionogene grenzflächenaktive Stoffe enthalten. Solche Verbindungen sind beispielsweise C₁₂-C₃₀-Fettsäuremono- und -diester von Anlagerungsprodukten von 1 bis 30 Mol Ethylenoxid an Glycerin; Polyglycerinester und alkoxylierte Polyglycerinester, wie beispielsweise Poly(3)glycerindiisostearat und Poly(2)glycerinpolyhydroxystearat; alkoxylierte, bevorzugt propoxylierte und insbesondere ethoxylierte, Mono- und Diglyceride, wie beispielsweise Glycerinmonolaurat + 20 EO (Mol Ethylenoxid) und Glycerinmonostearat + 20 EO; Aminoxide; Sorbitanfettsäureester und Anlagerungeprodukte von Ethylenoxid an Sorbitanfettsäureester wie beispielsweise Polysorbate, Sorbitanmonolaurat und Sorbitanmonolaurat + 20 EO; Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 0 bis 30 Ethylenoxid- und/oder Propylenoxid-Einheiten, wie beispielsweise Nonylphenol + 4 EO, Nonylphenol + 9 EO, Octylphenol + 3 EO und Octylphenol + 8 EO, sowie Alkylpolyglycoside. Als nichtionische Tenside eignen sich insbesondere Alkylmono- und -oligoglycoside und deren ethoxylierte Analoga. Bevorzugt sind solche Alkylpolyglykoside der Formel RO-(Z)ₓ, bei denen R im Wesentlichen aus C₈-C₁₈-Alkylgruppen besteht. Als Zuckerbaustein Z können beliebige Mono- oder Oligosaccharide, wie Glucose, Fructose, Galactose, Arabinose und Sucrose, eingesetzt werden. Glucose ist besonders bevorzugt. Die erfindungsgemäß verwendbaren Alkylpolyglykoside enthalten im Schnitt 1,1 bis 5 Zuckereinheiten. Die zusätzlichen nichtionischen Tenside werden in bevorzugt in einer Gesamtmenge von 0,1 bis 45 Gew.%, bevorzugt 1 bis 30 Gew.% und ganz besonders bevorzugt von 1 bis 20 Gew.%, bezogen auf die Gesamtmenge des anwendungsbereiten Mittels, eingesetzt.

Weiterhin können die Mittel kationische Tenside vom Typ der quartären Ammoniumverbindungen, der Esterquats und der Amidoamine, wie Stearamidopropyldimethylamin, enthalten. Bevorzugte quaternäre Ammoniumverbindungen sind Ammoniumhalogenide, wie Alkyltrimethylammoniumchloride, Dialkyldimethylammoniu chloride und Trialkylmethylammoniumchloride sowie die unter den INCI-Bezeichnungen Quaternium-27 und Quaternium-83 bekannten Verbindungen. Weitere erfindungsgemäß verwendbare kationische Tenside stellen die quaternisierten Proteinhydrolysate dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen (Warenzeichen Stepantex, Dehyquart und Armocare). Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, bevorzugt Dimethicon, Lichtschutzmittel, Proteine und Proteinhydrolysate, die gegebenenfalls geeignet modifiziert sind, Wirkstoffe wie Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die Mittel gemäß erstem Erfindungsgegenstand weisen bevorzugt einen pH-Wert im Bereich von 7 bis 11 auf, um die Farbstoffvorprodukte zu stabilisieren und eine stabile Gelbildung zu garantieren. Bei den pH-Werten im Sinne der vorliegenden Erfindung handelt es sich um pH-Werte, die bei einer Temperatur von 25 °C gemessen wurden.

Zur Einstellung des pH-Werts sind dem Fachmann gängige Acidifizierungs- und Alkalisierungsmittel geläufig. Die zur Einstellung des bevorzugten pH-Wertes erfindungsgemäß verwendbaren Alkalisierungsmittel können aus der Gruppe, die gebildet wird aus basischen Aminosäuren, Aminen, Ammoniak, Alkalimetallhydroxiden, Alkalimetallmetasilikaten, Alkalimetallphosphaten und Alkalimetallhydrogenphosphaten ausgewählt werden. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbemittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Die erfindungsgemäßen Färbemittel besitzen durch ihre spezifische Trägerbasis signifikante Vorteile gegenüber dem Stand der Technik im Hinblick auf ihre rheologischen Eigenschaften. Insbesondere zeichnen sich die erfindungsgemäßen Mittel durch eine hohe Lagerstabilität aus. Die Mittel behalten eine stabile Viskosität ohne wesentlichen Anstieg und insbesondere ohne Abfall in der Viskosität, was zu erheblichen Lager- und Anwendungsproblemen führen könnte. Diese Stabilität in der Viskosität gilt insbesondere für die Zubereitung, enthaltend die Oxidationsfarbstoffvorprodukte, aber auch für das anwendungsbereite Mittel nach Vermischen von Oxidationsmittelzubereitung und Zubereitung, enthaltend die Oxidationsfarbstoffvorprodukte.

Darüber hinaus lässt sich aufgrund der Transparenz des Trägers der Fortschritt des Färbevorgangs gut beobachten und bei gewünschten Färbeergebnis durch Ausspülen des verbliebenen Mittels anhalten. Auch führen die Färbungen mit den erfindungsgemäßen Mitteln zu intensiveren Färbeergebnissen als mit handelsüblichen Cremeformulierungen von Haarfärbemitteln. Dies führt auch dazu, dass für ein gleiches Färbeergebnis die Farbstoffmengen des erfindungsgemäßen Mittels gegenüber handelsüblichen Cremeformulierungen deutlich reduziert werden können.

Ein zweiter Gegenstand der vorliegenden Erfindung ist eine Mehrkomponentenverpackungseinheit (Kit-of-Parts), umfassend mindestens zwei getrennt voneinander konfektionierte Container, dadurch gekennzeichnet, dadurch gekennzeichnet, dass
A. ein Container (I) ein Mittel, enthaltend in einem gelförmigen Träger mindestens ein Oxidationsfarbstoffvorprodukt, wobei der gelförmige Träger des Mittels
   a) mindestens eine Emulgator-Kombination aus
      i) einem oder mehreren nichtionischen, polyethoxylierten Emulgatoren (i), ausgewählt aus ethoxylierten Fettalkoholen mit 8 bis 22 Kohlenstoffatomen und/oder ethoxylierten Rizinusöl und
      ii) einem oder mehreren Polyethylenglycol(en) (ii) mit einem mittleren Molekulargewicht von 100 bis 100000 g·Mol⁻¹
   b) und mindestens einen polymeren Verdicker, ausgewählt aus Homopolymeren von Acrylsäure oder Methacrylsäure und/oder Copolymeren von Acrylsäure oder Methacrylsäure mit C₁-C₄-Alkylestern von Acrylsäure oder Methacrylsäure, enthält,
   und
B. ein weiterer Container (II) eine Oxidationsmittelzubereitung, enthaltend in einem physiologisch verträglichen Träger mindestens Wasserstoffperoxid, beinhaltet.

Wird eine besonders starke Aufhellwirkung durch Einsatz von Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen. Unter Container wird im Rahmen der vorliegenden Erfindung eine Umhüllung verstanden, die in Form einer gegebenenfalls wieder-verschließbaren Flasche, einer Dose, eines Tütchens, eines Sachets oder ähnlichen Umhüllungen vorliegt. Dabei kann die Umhüllung aus Kunststoff, Glas, (Metall-) blech, Karton, Papier oder einem Verbundstoff gefertigt sein.

Weiterhin enthält die Mehrkomponentenverpackungseinheit (Kit-of-Parts) zusätzlich eine Gebrauchsleitung. Darüber hinaus kann es bevorzugt sein, wenn weiterhin eine Applikationshilfe, wie beispielsweise ein Kamm oder ein Pinsel oder eine Anmischschale, und/oder eine persönliche Schutzausrüstung, wie beispielsweise Einweg-Handschuhe dem Kit beigefügt ist.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der vorliegenden Erfindung ist ein Verfahren zum Färben menschlicher Haare, umfassend die Schritte:
A. Vermischen eines Mittel A mit einer Oxidationsmittelzubereitung, enthaltend in einem physiologisch verträglichen Träger mindestens Wasserstoffperoxid, unmittelbar vor der Anwendung;
B. Auftragen des anwendungsbereite Mittel auf das Haar;
C. Einwirken des Mittels für einen Zeitraum von 5 bis 45 Minuten, bevorzugt von 15 bis 35 Minuten, auf dem Haar;
D. Ausspülen des Haares;
dadurch gekennzeichnet, dass das Mittel A in einem gelförmigen Träger mindestens ein Oxidationsfarbstoffvorprodukt enthält und der gelförmige Träger
a) mindestens eine Emulgator-Kombination aus
   i) einem oder mehreren nichtionischen, polyethoxylierten Emulgatoren (i), ausgewählt aus ethoxylierten Fettalkoholen mit 8 bis 22 Kohlenstoffatomen und/oder ethoxylierten Rizinusöl und
   ii) einem oder mehreren Polyethylenglycol(en) (ii) mit einem mittleren Molekulargewicht von 100 bis 100000 g·Mol⁻¹
b) und mindestens einen polymeren Verdicker, ausgewählt aus Homopolymeren von Acrylsäure oder Methacrylsäure und/oder Copolymeren von Acrylsäure oder Methacrylsäure mit C₁-C₄-Alkylestern von Acrylsäure oder Methacrylsäure,
enthält.

Ein weiterer Gegenstand der vorliegenden Erfindung ist weiterhin ein Verfahren zum Färben menschlicher Haare, bei dem ein Mittel unmittelbar vor der Anwendung durch Vermischung der Komponenten der Mehrkomponentenverpackungseinheit des zweiten Erfindungsgegenstands hergestellt wird, das nunmehr anwendungsbereite Mittel auf das Haar aufgetragen wird, für eine Einwirkzeit von 2 bis 45 Minuten, bevorzugt von 15 bis 35 Minuten, auf dem Haar belassen wird, und anschließend das Haar ausgespült wird.

Die Anwendungstemperaturen beim erfindungsgemäßen Färbeverfahren können in einem Bereich zwischen 15 und 45 °C liegen. Nach der Einwirkungszeit wird das Haarfärbemittel durch Ausspülen, gegebenenfalls mit Hilfe eines Shampoos, von dem zu färbenden Haar entfernt. Das Nachwaschen mit einem Shampoo kann entfallen, wenn ein stark tensidhaltiger Träger, z. B. ein Färbeshampoo, verwendet wurde.

Bezüglich weiterer bevorzugter Ausführungsformen der erfindungsgemäßen Verfahren gilt mutatis mutandis das zu den erfindungsgemäßen Mitteln Gesagte.

Ein weiterer Gegenstand der Erfindung ist die Verwendung eines optisch transparenten, gelförmigen Trägers, enthaltend mindestens ein Oxidationsfarbstoffvorprodukt, zur Steuerung der bedarfsgerechten Einwirkzeit des Mittels beim Färben menschlicher Haare.

Durch Transparenz des Trägers lässt sich der Fortschritt des Färbevorgangs gut beobachten und bei gewünschten Färbeergebnis durch Ausspülen des verbliebenen Mittels anhalten, so dass die Einwirkzeit bedarfsgerecht bestimmbar ist.

In einer bevorzugten Ausführungsform dieses Erfindungsgegenstands enthält der gelförmige Träger neben Oxidationsfarbstoffvorprodukt(en) mindestens einen polymeren Verdicker, wie beispielsweise Polysaccharide oder (Meth)Acrylsäure(co)polymere. Besonders bevorzugt sind dabei gelförmige Träger, die einen polymeren Verdicker, ausgewählt aus Homopolymeren von Acrylsäure oder Methacrylsäure und/oder Copolymeren von Acrylsäure oder Methacrylsäure mit C₁-C₄-Alkylestern von Acrylsäure oder Methacrylsäure, enthalten.

In einer weiteren bevorzugten Ausführungsform dieses Erfindungsgegenstands enthält der gelförmige Träger neben Oxidationsfarbstoffvorprodukt(en) mindestens einen nichtionischen, polyethoxylierten Emulgator, bevorzugt ausgewählt aus ethoxylierten Fettalkoholen mit 8 bis 22 Kohlenstoffatomen und/oder ethoxylierten Rizinusöl.

In einer weiteren bevorzugten Ausführungsform dieses Erfindungsgegenstands enthält der gelförmige Träger neben Oxidationsfarbstoffvorprodukt(en) weiterhin mindestens ein Polyethylenglycol mit einem mittleren Molekulargewicht von 100 bis 100000 g·Mol⁻¹.

Zur Erhöhung der Transparenz der Träger sind dabei Kombinationen aus mindestens einem voranstehend beschriebenen nichtionischen, polyethoxylierten Emulgator und mindestens einem Polyethylenglycol mit einem mittleren Molekulargewicht von 100 bis 100000 g·Mol⁻¹ besonders bevorzugt.

Um die Stabilität und damit letztlich die Transparenz solcher gelförmigen Träger zu gewährleisten, sind gelförmige Träger, die
mindestens eine Emulgator-Kombination aus
i) einem oder mehreren nichtionischen, polyethoxylierten Emulgatoren (i), ausgewählt aus ethoxylierten Fettalkoholen mit 8 bis 22 Kohlenstoffatomen und/oder ethoxylierten Rizinusöl und
ii) einem oder mehreren Polyethylenglycol(en) (ii) mit einem mittleren Molekulargewicht von 100 bis 100000 g·Mol⁻¹
und mindestens einen polymeren Verdicker, ausgewählt aus Homopolymeren von Acrylsäure oder Methacrylsäure und/oder Copolymeren von Acrylsäure oder Methacrylsäure mit C₁-C₄-Alkylestern von Acrylsäure oder Methacrylsäure, enthalten, besonders bevorzugt.

Bezüglich weiterer bevorzugter Ausführungsformen der erfindungsgemäßen Verwendung gilt mutatis mutandis das zu den erfindungsgemäßen Mitteln Gesagte.

### Beispiele

Herstellung von Färbemitteln (Mengenangaben jeweils in prozentualen Gewichtsanteilen)

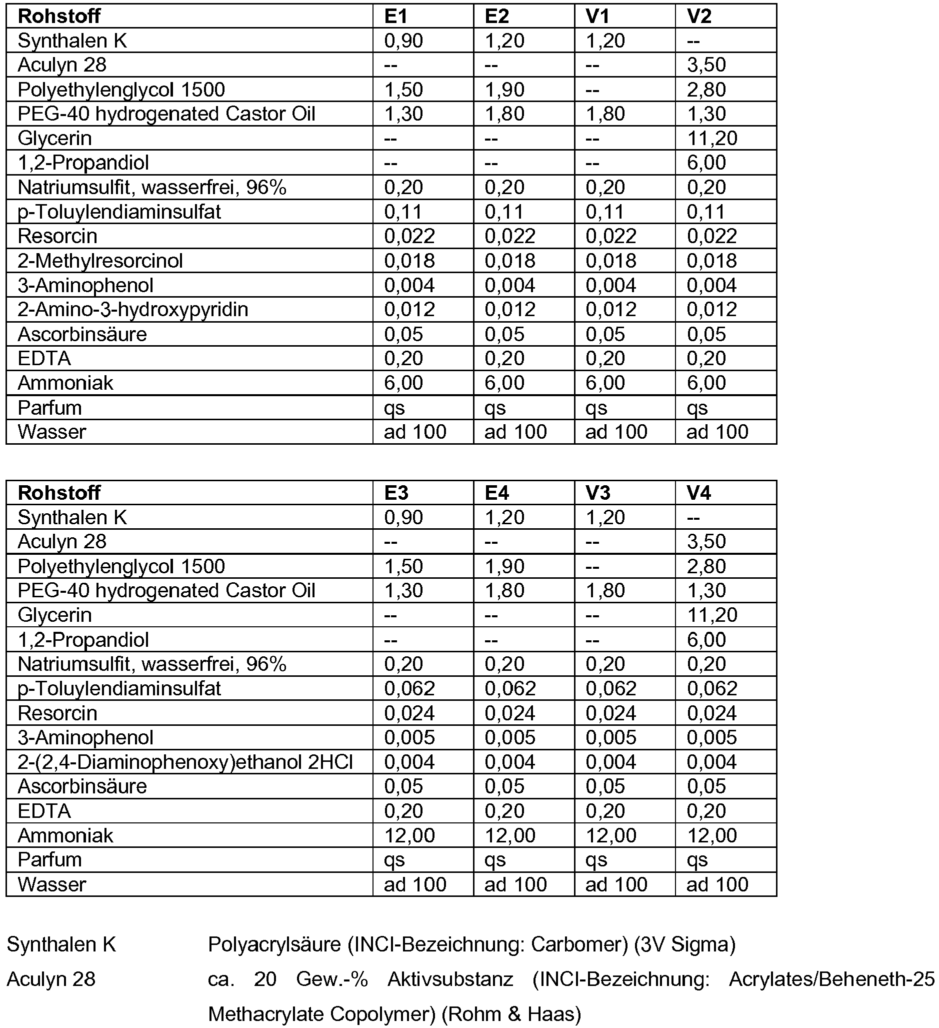

Die Mittel E2 und E4 zeichneten sich durch eine für die Anwendungsanforderungen geeignete Viskosität aus, während die Mittel E1 und E3 sowie V2 und V4 zwar noch eine akzeptable Viskosität aufwiesen, aber insgesamt geringfügig zu dünnflüssig waren. Während die erfindungsgemäßen Zubereitungen E1, E2, E3 und E4 sowie und die nicht erfindungsgemäßen Mittel V2 und V4 klare Gele bildeten, waren die Mittel V1 und V3 jeweils trübe und damit zur Nachverfolgung einer Färbereaktion ungeeignet.

Die Färbemittel E1, E2, E3 und E4 sowie V1, V2, V3 und V4 wurden vor der Anwendung jeweils mit einer Entwicklerzubereitung EZ im Verhältnis 1 : 1 vermischt.

| **Rohstoff** | **EZ** |
|---|---|
| NaOH, 45 Gew.-% | 0,73 |
| Dipicolinsäure | 0,10 |
| Dinatrium Pyrophosphat | 0,03 |
| Etidronsäure, 60 Gew.-% | 1,50 |
| 1,2-Propandiol | 4,00 |
| Xanthan Gum | 2,00 |
| Wasserstoffperoxid, 50 Gew.-% | 18,20 |
| Wasser | ad 100 |

Es zeigte sich, dass die erfindungsgemäßen Anwendungsmischungen E1/EZ, E2/EZ, E3/EZ und E4/EZ sich einerseits gut durch Verrühren anmischen ließen und eine klare, gelförmige Färbezubereitung ergaben, wobei die Anwendungsmischungen E2/EZ sowie E4/EZ eine optimale Viskosität zur Anwendung aufwiesen. Die Kombinationen aus V1/EZ, V2/EZ, V3/EZ und V4/EZ ließen sich nur unzureichend zu geeigneten Anwendungsmischungen miteinander verrühren.

## Patentansprüche

1. Mittel zum Färben keratinischer Fasern, insbesondere menschlicher Haare, enthaltend in einem gelförmigen Träger mindestens ein Oxidationsfarbstoffvorprodukt, **dadurch gekennzeichnet, dass** das Mittel frei von Fettstoffen ist, und
der gelförmige Träger des Färbemittels
a) mindestens eine Emulgator-Kombination aus
i) einem oder mehreren nichtionischen, polyethoxylierten Emulgatoren (i), ausgewählt aus ethoxylierten Fettalkoholen mit 8 bis 22 Kohlenstoffatomen und/oder ethoxylierten Rizinusöl
und
ii) einem oder mehreren Polyethylenglycol(en) (ii) mit einem mittleren Molekulargewicht von 100 bis 100000 g·Mol⁻¹
b) und mindestens einen polymeren Verdicker, ausgewählt aus Homopolymeren von Acrylsäure oder Methacrylsäure und/oder Copolymeren von Acrylsäure oder Methacrylsäure mit C₁-C₄-Alkylestern von Acrylsäure oder Methacrylsäure,
enthält.

2. Mittel nach Anspruch 1, **dadurch gekennzeichnet, dass** der gelförmige Träger optisch transparent ist.

3. Mittel nach Anspruch 1 oder 2, **dadurch gekennzeichnet, dass** es frei von Fettalkoholen, hydrophoben Fettsäureestern sowie mineralischen Fettstoffen ist.

4. Mittel nach einem der Ansprüche 1 bis 3 , **dadurch gekennzeichnet, dass** es als nichtionischen, polyethoxylierten Emulgator (i) ethoxyliertes Rizinusöl mit einem durchschnittlichen Ethoxylierungsgrad von 20 bis 150, bevorzugt 30 bis 100 und besonders bevorzugt 35 bis 80 enthält.

5. Mittel nach einem der Ansprüche 1 bis 4, **dadurch gekennzeichnet, dass** es als nichtionischen, polyethoxylierten Emulgator (i) PEG-40 hydrogenated Castor Oil enthält.

6. Mittel nach einem der Ansprüche 1 bis 5, **dadurch gekennzeichnet, dass** es als Polyethylenglycol (ii) ein Polyethylenglycol mit einem mittleren Molekulargewicht von 500 bis 15000 g·Mol⁻¹, bevorzugt 1000 bis 10000 g·Mol⁻¹ und besonders bevorzugt 1200 bis 5000 g·Mol⁻¹ enthält.

7. Mittel nach einem der Ansprüche 1 bis 6, **dadurch gekennzeichnet, dass** es als Polyethylenglycol(e) (ii) PEG-30, PEG-32, PEG-35, PEG-38, PEG-40, PEG-45, PEG-50, PEG-60, PEG-75, PEG-80, PEG-100 (d.h. Ethoxylierungsgrad 30, 32, 35, 38, 40, 50 etc.) oder PEG 1000, PEG 1200, PEG 1500, PEG 2000, PEG 3000, PEG 5000, PEG 6000 (d.h. mittleres Molgewicht 1000, 1200, 1500, 2000 etc.) enthält.

8. Mittel nach einem der Ansprüche 1 bis 7, **dadurch gekennzeichnet, dass** die Emulgator-Kombination a) ein Gewichtsverhältnis aus dem Anteil der nichtionischen, polyethoxylierten Emulgatoren (i) und dem Anteil des Polyethylenglycols (ii) einen Wert von 3:1 bis 1:3, bevorzugt von 2:1 bis 1:2 und insbesondere von 1,5:1 bis 1:1,5, aufweist.

9. Mittel nach einem der Ansprüche 1 bis 8, **dadurch gekennzeichnet, dass** die Emulgator-Kombination in einem Anteil von 0,5 bis 15,0 Gew.-%, bevorzugt 1,0 bis 10,0 Gew.-%, besonders bevorzugt 1,5 bis 7,5 Gew.-% und insbesondere 3,0 bis 5,0 Gew.-%, bezogen auf das Gesamtgewicht des Mittels, enthalten ist.

10. Mittel nach einem der Ansprüche 1 bis 9, **dadurch gekennzeichnet, dass** es als polymeren Verdicker Polyacrylsäure (Carbomer) enthält.

11. Mittel nach einem der Ansprüche 1 bis 10, **dadurch gekennzeichnet, dass** der polymere Verdicker von 0,1 bis 5,0 Gew.-%, bevorzugt 0,3 bis 3,0 Gew.-%, besonders bevorzugt 0,5 bis 2,5 Gew.-% und insbesondere 1,0 bis 2,0 Gew.-%, bezogen auf das Gesamtgewicht des Mittels, enthalten ist.

12. Mittel nach einem der Ansprüche 1 bis 11, **dadurch gekennzeichnet, dass** es einen gelförmigen Träger aufweist, der 0,2 bis 2,5 Gew.-% nichtionische, polyethoxylierte Emulgatoren (i), 0,2 bis 2,5 Gew.-% Polyethylenglycole (ii) und 0,2 bis 2,0 Gew.-% polymeren Verdicker, jeweils bezogen auf das Gesamtgewicht des Mittels, enthält.

13. Mehrkomponentenverpackungseinheit (Kit-of-Parts), umfassend mindestens zwei getrennt voneinander konfektionierte Container, **dadurch gekennzeichnet, dass**
A. ein Container (I) ein Mittel nach einem der Ansprüche 1 bis 12 enthält und
B. ein Container (II) eine Oxidationsmittelzubereitung, enthaltend in einem physiologisch verträglichen Träger mindestens Wasserstoffperoxid,
beinhaltet.

14. Verfahren zum Färben menschlicher Haare, umfassend die Schritte:
A. Vermischen eines Mittel nach einem der Ansprüche 1 bis 12 mit einer Oxidationsmittelzubereitung, enthaltend in einem physiologisch verträglichen Träger mindestens Wasserstoffperoxid, unmittelbar vor der Anwendung;
B. Auftragen des anwendungsbereite Mittel auf das Haar;
C. Einwirken des Mittels für einen Zeitraum von 5 bis 45 Minuten, bevorzugt von 15 bis 35 Minuten, auf dem Haar;
D. Ausspülen des Haares.

## Claims

1. An agent for dyeing keratinic fibers, in particular human hair, containing in a gel-like carrier at least one oxidation dye precursor, **characterized in that** the agent is free of fatty substances and the gel-like carrier of the dye contains:
a) at least one emulsifier combination of:
i) one or more non-ionic polyethoxylated emulsifiers (i) selected from ethoxylated fatty alcohols having from 8 to 22 carbon atoms and/or ethoxylated castor oil, and
ii) one or more polyethylene glycol(s) (ii) having an average molecular weight of from 100 to 100,000 g·Mol⁻¹,
b) and at least one polymeric thickener selected from homopolymers of acrylic acid or methacrylic acid and/or copolymers of acrylic acid or methacrylic acid having C₁-C₄ alkyl esters of acrylic acid or methacrylic acid.

2. The agent according to claim 1, **characterized in that** the gel-like carrier is optically transparent.

3. The agent according to claim 1 or 2, **characterized in that** said agent is free of fatty alcohols, hydrophobic fatty acid esters and mineral fatty substances.

4. The agent according to one of claims 1 to 3, **characterized in that** said agent contains ethoxylated castor oil having an average degree of ethoxylation of from 20 to 150, preferably from 30 to 100, and particularly preferably from 35 to 80, as the non-ionic polyethoxylated emulsifier (i).

5. The agent according to one of claims 1 to 4, **characterized in that** said agent contains PEG-40 hydrogenated castor oil as the non-ionic polyethoxylated emulsifier (i).

6. The agent according to one of claims 1 to 5, **characterized in that** said agent contains a polyethylene glycol having an average molecular weight of from 500 to 15,000 g·Mol⁻¹, preferably from 1,000 to 10,000 g·Mol⁻¹, and particularly preferably from 1,200 to 5,000 g·Mol⁻¹, as the polyethylene glycol (ii).

7. The agent according to one of claims 1 to 6, **characterized in that** said agent contains PEG-30, PEG-32, PEG-35, PEG-38, PEG-40, PEG-45, PEG-50, PEG-60, PEG-75, PEG-80, PEG-100 (i.e. degree of ethoxylation 30, 32, 35, 38, 40, 50, etc.) or PEG 1000, PEG 1200, PEG 1500, PEG 2000, PEG 3000, PEG 5000, PEG 6000 (i.e. average molecular weight 1,000, 1,200, 1,500, 2,000, etc.) as the polyethylene glycol(s) (ii).

8. The agent according to one of claims 1 to 7, **characterized in that** the emulsifier combination a) has a weight ratio of the proportion of non-ionic polyethoxylated emulsifiers (i) to the proportion of the polyethylene glycol (ii) of from 3:1 to 1:3, preferably of from 2:1 to 1:2, and in particular of from 1.5:1 to 1:1.5.

9. The agent according to one of claims 1 to 8, **characterized in that** the emulsifier combination is contained in a proportion of from 0.5 to 15.0 wt.%, preferably from 1.0 to 10.0 wt.%, particularly preferably from 1.5 to 7.5 wt.%, and in particular from 3.0 to 5.0 wt.%, based on the total weight of the agent.

10. The agent according to one of claims 1 to 9, **characterized in that** said agent contains a polyacrylic acid (carbomer) as the polymeric thickener.

11. The agent according to one of claims 1 to 10, **characterized in that** the polymeric thickener is contained in an amount of from 0.1 to 5.0 wt.%, preferably from 0.3 to 3.0 wt.%, particularly preferably from 0.5 to 2.5 wt.%, and in particular from 1.0 to 2.0 wt.%, based on the total weight of the agent.

12. The agent according to one of claims 1 to 11, **characterized in that** said agent comprises a gel-like carrier which contains from 0.2 to 2.5 wt.% non-ionic polyethoxylated emulsifiers (i), from 0.2 to 2.5 wt.% polyethylene glycols (ii), and from 0.2 to 2.0 wt.% polymeric thickener, in each case based on the total weight of the agent.

13. A multi-component packaging unit (kit-of-parts), comprising at least two containers that are manufactured separately from one another, **characterized in that**
A. one container (I) contains an agent according to one of claims 1 to 12 and
B. one container (II) contains an oxidizing agent preparation containing, in a physiologically acceptable carrier, at least hydrogen peroxide.

14. A method for dyeing human hair, comprising the steps of:
A. mixing an agent according to one of claims 1 to 12 with an oxidizing agent preparation containing, in a physiologically acceptable carrier, at least hydrogen peroxide, immediately before use;
B. applying the ready-to-use agent onto the hair;
C. allowing the agent to act on the hair for a period of from 5 to 45 minutes; preferably from 15 to 35 minutes;
D. rinsing the hair.

## Revendications

1. Produit de coloration de fibres de kératine, en particulier de cheveux humains, contenant, dans un vecteur en gel, au moins un précurseur de coloration d'oxydation, **caractérisé en ce que** le produit ne contient pas de graisses, et
le vecteur en gel du produit de coloration contient :
a) au moins une combinaison d'émulsifiants constituée de
i) un ou plusieurs émulsifiants non-ioniques polyéthoxylés (i), choisi parmi des alcools gras éthoxylés avec 8 à 22 atomes de carbone et/ou de l'huile de ricin éthoxylé, et
ii) un ou plusieurs polyéthylènes glycols (ii) avec un poids moléculaire moyen de 100 à 100 000 g.mol⁻¹
b) au moins un épaississant polymère choisi parmi des homopolymères d'acide acrylique ou méthacrylique et/ou de copolymères d'acide acrylique ou méthacrylique avec des esters de C₁₋₄-alkyle d'acide acrylique ou méthacrylique.

2. Produit selon la revendication 1, **caractérisé en ce que** le vecteur en gel est optiquement transparent.

3. Produit selon la revendication 1 ou 2, **caractérisé en ce qu'**il est exempt d'alcools gras, esters hydrophobes d'acides gras, ainsi que d'acides gras minéraux.

4. Produit selon une des revendications 1 à 3, **caractérisé en ce qu'**il contient comme émulsifiant non-ionique polyéthoxylé (i) de l'huile de ricin éthoxylé avec un degré d'éthoxylation moyen de 20 à 150, préférentiellement de 30 à 100 et particulièrement préférentiellement de 35 à 80.

5. Produit selon une des revendications 1 à 4, **caractérisé en ce qu'**il contient comme émulsifiant non-ionique polyéthoxylé (i) de l'huile de ricin hydrogénée PEG-40.

6. Produit selon une des revendications 1 à 5, **caractérisé en ce qu'**il contient comme polyéthylène glycol (ii) un polyéthylène de poids moléculaire moyen de 500 à 15 000 g.Mol⁻¹, préférentiellement de 1 000 à 10 000 g.Mol⁻¹ et particulièrement préférentiellement de 1 200 à 5 000 g.mol⁻¹.

7. Produit selon une des revendications 1 à 6, **caractérisé en ce qu'**il contient comme polyéthylène glycol (ii) PEG-30, PEG-32, PEG-35, PEG-38, PEG-40, PEG-45, PEG-50, PEG-60, PEG-75, PEG-80, PEG-100 (c'est-à-dire degré d'éthoxylation 30, 32, 35, 38, 40, 50, etc.) ou PEG 1000, PEG 1200, PEG 1500, PEG 2000, PEG 3000, PEG 5000, PEG 6000 (c'est-à-dire poids moléculaire moyen 1 000, 1 200, 2 000, etc.)

8. Produit selon une des revendications 1 à 7, **caractérisé en ce que** la combinaison d'émulsifiants a) présente un rapport pondéral de la portion des émulsifiants non-ioniques polyéthoxylés (i) et de la portion des polyéthylènes glycols (ii) de 3:1 à 1:3, préférentiellement de 2:1 à 1:2, et en particulier de 1,5:1 à 1:1,5.

9. Produit selon une des revendications 1 à 8, **caractérisé en ce que** la combinaison d'émulsifiants est contenue à hauteur de 0,5 à 15,0 % en poids, préférentiellement de 1,0 à à 10,0 % en poids, particulièrement préférentiellement de 1,5 à 7,5 % en poids et en particulier de 3,0 à 5,0 % en poids rapporté au poids total du produit.

10. Produit selon une des revendications 1 à 9, **caractérisé en ce qu'**il contient comme épaississant polymère du polyacide acrylique (carbomère).

11. Produit selon une des revendications 1 à 10, **caractérisé en ce que** l'épaississant polymère est contenu à hauteur de 0,1 à 5,0 % en poids, préférentiellement de 0,3 à 3,0 % en poids, particulièrement préférentiellement de 0,5 à 2,5 % en poids et en particulier de 1,0 à 2,0 % en poids rapporté au poids total du produit.

12. Produit selon une des revendications 1 à 11, **caractérisé en ce qu'**il contient un vecteur en gel qui contient de 0,2 à 2,5 % en poids d'émulsifiants polyéthoxylés non-ioniques (i), de 0,2 à 2,5 % en poids de polyéthylènes glycols (ii) et de 0,2 à 2,0 % en poids d'épaississants polymères, respectivement rapportés au poids total du produit.

13. Unité d'emballage de plusieurs composants (kit) comprenant au moins deux récipients confectionnés séparés l'un de l'autre, **caractérisée en ce que** :
A. un récipient (I) contient un produit selon une des revendications 1 à 12, et
B. un récipient (II) contient une préparation de produit d'oxydation comprenant, dans un vecteur physiologiquement compatible, au moins du peroxyde d'hydrogène.

14. Procédé de coloration de cheveux humains, comprenant les étapes de :
A. mélange d'un produit selon une des revendications 1 à 12 avec une préparation de produit d'oxydation contenant, dans un vecteur physiologiquement compatible, au moins du peroxyde d'hydrogène, immédiatement avant utilisation ;
B. application du produit prêt à l'emploi sur les cheveux ;
C. action du produit pendant une période de 5 à 45 minutes, de préférence de 15 à 35 minutes, sur les cheveux ;
D. rinçage des cheveux.
